(19) Europäisches Patentamt
European Patent Office
Office européen des brevets

(11) Veröffentlichungsnummer: **0 467 139 A2**

# (12) EUROPÄISCHE PATENTANMELDUNG

(21) Anmeldenummer: **91110831.4**

(22) Anmeldetag: **29.06.91**

(51) Int. Cl.5: **C07D 401/12**, A01N 43/54, A01N 43/66, C07D 213/84

(30) Priorität: **14.07.90 DE 4022478**

(43) Veröffentlichungstag der Anmeldung: **22.01.92 Patentblatt 92/04**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Heinemann, Ulrich, Dr.**
**Am Sonnenhang 1**
**W-5653 Leichlingen 2(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Schmidt, Robet R., Dr.**
**Im Waldwinkel 110**
**W-5060 Bergisch Gladbach 2(DE)**

## (54) Nicotinsäurederivate als Herbizide.

(57) Beschrieben werden neue Nicotinsäurederivate der Formel (I)

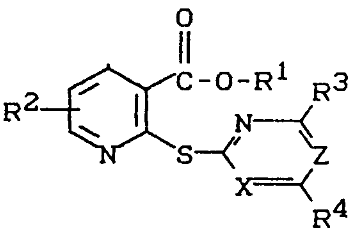

(I)

in der $R^1$, $R^2$, $R^3$, $R^4$, X und Z die in der Beschreibung angegebene Bedeutung haben sowie ein Verfahren zu deren Herstellung.

Die neuen Nicotinsäurederivate der Formel (I) werden als Herbizide verwendet.

EP 0 467 139 A2

Die Erfindung betrifft neue Nicotinsäurederivate, ein Verfahren zu ihrer Herstellung und ihre Verwendung als Herbizide.

Es ist bekannt, daß bestimmte substituierte Benzoesäurederivate, wie beispielsweise die Verbindung 2-[2-(4,6-dimethoxy)-pyrimidinylthio]-benzoesäuremethylester herbizide Eigenschaften besitzen (vgl. z.B. EP 223406).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Nicotinsäurederivate der allgemeinen Formel (I),

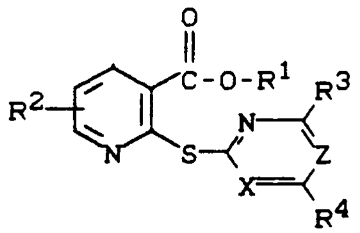

(I)

in welcher

| | |
|---|---|
| $R^1$ | für Wasserstoff, Alkyl, für ein Alkalimetallkation oder für ein gegebenenfalls substituiertes Ammoniumion steht, |
| $R^2$ | für Wasserstoff, Halogen, Alkyl, Alkoxy oder Halogenalkyl steht, |
| $R^3$ und $R^4$ | unabhängig voneinander jeweils für Wasserstoff, Alkyl, Alkoxy, Alkylthio, Halogenalkyl, Halogenalkoxy oder Halogenalkylthio stehen, |
| X | für Stickstoff oder für einen Rest C-$R^5$ steht und |
| Z | für Stickstoff oder für einen Rest C-$R^6$ steht, wobei |
| $R^5$ | für Wasserstoff oder Cyano steht und |
| $R^6$ | für Wasserstoff oder Alkyl steht, |

gefunden.

Weiterhin wurde gefunden, daß man die neuen Nicotinsäurederivate der allgemeinen Formel (I),

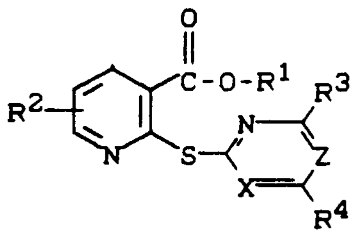

(I)

in welcher

| | |
|---|---|
| $R^1$ | für Wasserstoff, Alkyl, für ein Alkalimetallkation oder für ein gegebenenfalls substituiertes Ammoniumion steht, |
| $R^2$ | für Wasserstoff, Halogen, Alkyl, Alkoxy oder Halogenalkyl steht, |
| $R^3$ und $R^4$ | unabhängig voneinander jeweils für Wasserstoff, Alkyl, Alkoxy, Alkylthio, Halogenalkyl, Halogenalkoxy oder Halogenalkylthio stehen, |
| X | für Stickstoff oder für einen Rest C-$R^5$ steht und |
| Z | für Stickstoff oder für einen Rest C-$R^6$ steht, wobei |
| $R^5$ | für Wasserstoff oder Cyano steht und |
| $R^6$ | für Wasserstoff oder Alkyl steht, |

erhält, wenn man Thiopyridoncarbonsäurederivate der Formel (II),

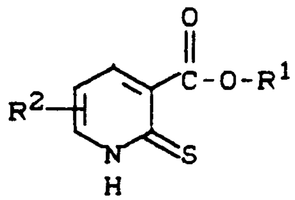

(II)

in welcher

$R^1$ und $R^2$ die oben angegebene Bedeutung haben,

mit Heterocyclen der Formel (III)

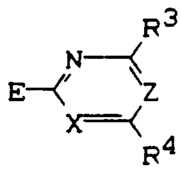

(III)

in welcher

E für eine elektronenanziehende Abgangsgruppe steht und

$R^3$, $R^4$, X und Z die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen Nicotinsäurederivate der Formel (I) gute herbizide, insbesondere auch selektiv herbizide Eigenschaften besitzen.

Überraschenderweise zeigen die erfindungsgemäßen Nicotinsäurederivate der allgemeinen Formel (I) eine deutlich höhere herbizide Wirksamkeit gegenüber Unkräutern bei vergleichbar guter Nutzpflanzenselektivität im Vergleich zu den aus dem Stand der Technik bekannten substituierten Benzoesäurederivaten, wie beispielsweise die Verbindung 2-[2-(4,6-Dimethoxypyrimidinyl)-thiol-benzoesäuremethylester, welche chemisch und wirkungsmäßig naheliegende Verbindungen sind.

Die erfindungsgemäßen Nicotinsäurederivate sind durch die Formel (I) allgemein definiert.

Bevorzugte Substituenten bzw. Bereiche der in den oben und nachstehend erwähnten Formeln aufgeführten Reste werden im folgenden erläutert:

Alkyl steht einzeln oder in zusammengesetzten Resten für geradkettiges oder verzweigtes Alkyl mit 1 bis 4 und vorzugsweise mit 1 oder 2 Kohlenstoffatomen; beispielhaf und vorzugsweise seien genannt Methyl, Ethyl, n-und i-Propyl, n-, i-, s- und t-Butyl.

Alkoxy und Alkylthio stehen in den allgemeinen Formeln für geradkettiges oder verzweigtes Alkoxy bzw. Alkylthio mit 1 bis 4 und bevorzugt 1 oder 2 Kohlenstoffatomen; beispielhaft und vorzugsweise seien genannt: Methoxy, Ethoxy, n- und i-Propoxy, n-, i-, s- und t-Butoxy, Methylthio, Ethylthio, n- und i-Propylthio, n-, i-, s- und t-Butylthio.

Halogenalkyl, Halogenalkoxy und Halogenalkylthio stehen in den allgemeinen Formeln für geradkettiges oder verzweigtes Halogenalkyl, Halogenalkoxy bzw. Halogenalkylthio mit 1 bis 4 und bevorzugt mit 1 oder 2 Kohlenstoffatomen und jeweils 1 bis 9 und bevorzugt 1 bis 5, gleichen oder verschiedenen Halogenatomen wie unter Halogen definiert; beispielhaft und vorzugsweise seien genannt: Fluormethyl, Chlormethyl, Brommethyl, Fluorethyl, Chlorethyl, Bromethyl, Fluor-n-propyl, Chlor-n-propyl, Dichlormethyl, Difluorethyl, Trifluorethyl, Trichlorethyl, Trifluorchlorethyl, Chlorbutyl, Fluorbutyl und insbesondere Difluormethyl, Trifluormethyl, Trichlormethyl, Dichlorfluor-methyl und Chlordifluor-methyl sowie die diesen entsprechenden Halogenalkoxyreste bzw. Halogenalkylthioreste.

Halogen steht im allgemeinen als Substituent oder in den Resten Halogenalkyl, Halogenalkoxy und Halogenalkylthio für Fluor, Chlor, Brom und Iod, insbesondere für Fluor und Chlor.

Ammoniumion steht in den allgemeinen Formeln vorzugweise für ein unsubstituiertes oder ein- bis dreifach, gleich oder verschieden durch geradkettiges oder verzweigtes Alkyl mit 1 bis 4,insbesondere 1 oder 2, Kohlenstoffatomen substituiertes Ammoniumkation. Beispielhaft und vorzugsweise seien genannt: Ammoniumkation und Triethylammoniumkation.

Alkalimetallkation steht in den allgemeinen Formeln vorzugsweise für ein Lithium-, insbesondere für ein Natrium- oder Kaliumkation.

Die hier aufgeführten Definitionen gelten in entsprechender Weise auch für die im folgenden aufgeführten bevorzugten Kombinationen von Resten.

Bevorzugt sind Verbindungen der Formel (I), bei welchen

| | |
|---|---|
| $R^1$ | für Wasserstoff, für geradkettiges oder verzweigtes Alkyl mit 1 bis 4 Kohlenstoffatomen, für ein Lithium-, Natrium- oder Kalium-Kation oder für ein gegebenenfalls ein- bis dreifach gleich oder verschieden durch geradkettiges oder verzweigtes Alkyl mit 1 bis 4 Kohlenstoffatomen substituiertes Ammoniumkation steht, |
| $R^2$ | für Wasserstoff, Fluor, Chlor, Brom, Iod, für jeweils geradkettiges oder verzweigtes Alkyl oder Alkoxy mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht, |
| $R^3$ und $R^4$ | unabhängig voneinander jeweils für Wasserstoff, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen stehen, |
| X | für Stickstoff oder für einen Rest C-$R^5$ steht und |
| Z | für Stickstoff oder für einen Rest C-$R^6$ steht, wobei |
| $R^5$ | für Wasserstoff oder Cyano steht und |
| $R^6$ | für Wasserstoff oder für geradkettiges oder verzweigtes Alkyl mit 1 bis 4 Kohlenstoffatomen steht. |

Besonders bevorzugt sind Verbindungen der Formel (I), bei welchen

| | |
|---|---|
| $R^1$ | für Wasserstoff, Methyl, Ethyl, n- oder i-Propyl oder für ein Lithium-, Natrium-, Kalium-, Ammonium- oder Triethylammoniumion steht, |
| $R^2$ | für Wasserstoff, Fluor, Chlor, Brom, Methyl, Ethyl, n- oder i-Propyl, Methoxy, Ethoxy, Trifluormethyl oder Trichlormethyl steht, |
| $R^3$ und $R^4$ | für Wasserstoff, Methyl, Ethyl, n- oder i-Propyl, Methoxy, Ethoxy, Methylthio, Ethylthio, Trifluormethyl, Trichlormethyl, Difluormethyl, Trifluormethoxy, Difluormethoxy, Trifluormethylthio, Trichlormethylthio, Difluorchlormethylthio oder Dichlorfluormethylthio stehen, |
| X | für Stickstoff oder eine C-CN-Gruppe steht und |
| Z | für Stickstoff oder eine C-$CH_3$- oder eine CH-Gruppe steht. |

Im einzelnen seien außer den bei den Herstellungsbeispielen genannten Verbindungen die folgenden Nicotinsäurederivate der allgemeinen Formel (I) genannt:

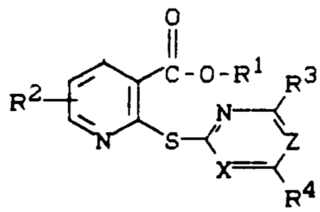

(I)

| $R^1$ | $R^2$ | $R^3$ | $R^4$ | X | Z |
|---|---|---|---|---|---|
| H | H | $CH_3O$ | $CH_3$ | N | CH |
| H | H | $CH_3$ | $CH_3$ | N | CH |
| H | H | $CH_3$ | $CH_3$ | N | $C-CH_3$ |
| H | $4-CH_3$ | $CH_3O$ | $CH_3O$ | N | CH |
| H | $5-CH_3$ | $CH_3O$ | $CH_3O$ | N | CH |
| H | $6-CH_3$ | $CH_3O$ | $CH_3O$ | N | CH |
| $CH_3$ | H | $CH_3O$ | $CH_3O$ | N | CH |
| $C_2H_5$ | H | $CH_3O$ | $CH_3O$ | N | CH |
| $Na^{\oplus}$ | H | $CH_3O$ | $CH_3O$ | N | CH |
| $K^{\oplus}$ | H | $CH_3O$ | $CH_3O$ | N | CH |
| $NH_4^{\oplus}$ | H | $CH_3O$ | $CH_3O$ | N | CH |
| $NH(C_2H_5)_3^{\oplus}$ | H | $CH_3O$ | $CH_3O$ | N | CH |
| $Li^{\oplus}$ | H | $CH_3O$ | $CH_3O$ | N | CH |
| $CH_3$ | H | $CH_3O$ | $CH_3$ | N | CH |
| $C_2H_5$ | H | $CH_3O$ | $CH_3$ | N | CH |
| $Na^{\oplus}$ | H | $CH_3O$ | $CH_3$ | N | CH |
| $K^{\oplus}$ | H | $CH_3O$ | $CH_3$ | N | CH |

| $R^1$ | $R^2$ | $R^3$ | $R^4$ | X | Z |
|---|---|---|---|---|---|
| $NH_4^{\oplus}$ | H | $CH_3O$ | $CH_3$ | N | CH |
| $NH(C_2H_5)_3^{\oplus}$ | H | $CH_3O$ | $CH_3$ | N | CH |
| H | 4-Cl | $CH_3O$ | $CH_3O$ | N | CH |
| H | 5-Cl | $CH_3O$ | $CH_3O$ | N | CH |
| H | 6-Cl | $CH_3O$ | $CH_3O$ | N | CH |
| $CH_3$ | H | $CH_3$ | $CH_3$ | N | CH |
| $C_2H_5$ | H | $CH_3$ | $CH_3$ | N | CH |
| $Na^{\oplus}$ | H | $CH_3$ | $CH_3$ | N | CH |
| $K^{\oplus}$ | H | $CH_3$ | $CH_3$ | N | CH |
| $NH_4^{\oplus}$ | H | $CH_3$ | $CH_3$ | N | CH |
| $NH(C_2H_5)_3^{\oplus}$ | H | $CH_3$ | $CH_3$ | N | CH |
| $CH_3$ | H | $CH_3$ | $CH_3$ | N | $C-CH_3$ |
| $C_2H_5$ | H | $CH_3$ | $CH_3$ | N | $C-CH_3$ |
| $Na^{\oplus}$ | H | $CH_3$ | $CH_3$ | N | $C-CH_3$ |
| $K^{\oplus}$ | H | $CH_3$ | $CH_3$ | N | $C-CH_3$ |
| H | $4-CH_3$ | $CH_3O$ | $CH_3O$ | N | N |
| H | $5-CH_3$ | $CH_3O$ | $CH_3O$ | N | N |
| H | $6-CH_3$ | $CH_3O$ | $CH_3O$ | N | N |
| H | 4-Cl | $CH_3O$ | $CH_3O$ | N | N |
| H | 5-Cl | $CH_3O$ | $CH_3O$ | N | N |
| H | 6-Cl | $CH_3O$ | $CH_3O$ | N | N |
| $CH_3$ | H | $CH_3O$ | $CH_3O$ | N | N |

| $R^1$ | $R^2$ | $R^3$ | $R^4$ | X | Z |
|---|---|---|---|---|---|
| $C_2H_5$ | H | $CH_3O$ | $CH_3O$ | N | N |
| $Na^{\oplus}$ | H | $CH_3O$ | $CH_3O$ | N | N |
| $K^{\oplus}$ | H | $CH_3O$ | $CH_3O$ | N | N |
| $NH_4^{\oplus}$ | H | $CH_3O$ | $CH_3O$ | N | N |
| $NH(C_2H_5)_3^{\oplus}$ | H | $CH_3O$ | $CH_3O$ | N | N |
| $Li^{\oplus}$ | H | $CH_3O$ | $CH_3O$ | N | N |
| H | 4-$C_2H_5$ | $CH_3$ | $CH_3$ | C-CN | N |
| H | 5-$OCH_3$ | $CH_3$ | $CH_3$ | C-CN | N |
| H | 6-$OC_2H_5$ | $CH_3$ | $CH_3$ | C-CN | N |
| H | 4-F | $CH_3$ | $CH_3$ | C-CN | N |
| H | 6-Br | $CH_3$ | $CH_3$ | C-CN | N |
| $CH_3$ | H | $CH_3$ | $CH_3$ | C-CN | N |
| $C_2H_5$ | H | $CH_3$ | $CH_3$ | C-CN | N |
| $Na^{\oplus}$ | H | $CH_3$ | $CH_3$ | C-CN | N |
| $K^{\oplus}$ | H | $CH_3$ | $CH_3$ | C-CN | N |
| $NH_4^{\oplus}$ | H | $CH_3$ | $CH_3$ | C-CN | N |
| H | 5-$CF_3$ | $CH_3$ | $CH_3$ | C-CN | N |
| H | H | $OC_2H_5$ | $OC_2H_5$ | N | CH |
| H | H | $OC_2H_5$ | $OC_2H_5$ | N | CH |
| H | H | $OC_2H_5$ | $OC_2H_5$ | N | CH |
| H | H | $SCH_3$ | $SCH_3$ | N | CH |
| H | H | $SC_2H_5$ | $SC_2H_5$ | N | CH |

| $R^1$ | $R^2$ | $R^3$ | $R^4$ | X | Z |
|---|---|---|---|---|---|
| H | H | $OCH_3$ | $CF_3$ | N | CH |
| H | H | $OCH_3$ | $CCl_3$ | N | CH |
| H | H | $OCH_3$ | $CHF_2$ | N | CH |
| H | H | $CH_3$ | $CF_3$ | N | CH |
| H | H | $CH_3$ | $CCl_3$ | N | CH |
| H | H | $CH_3$ | $CHF_2$ | N | CH |
| H | H | $OC_2H_5$ | $OC_2H_5$ | N | N |
| H | H | $OC_2H_5$ | $CH_3$ | N | $C-CH_3$ |
| H | H | $OCH_3$ | $CH_3$ | N | $C-CH_3$ |

Verwendet man beispielsweise 2-Mercaptonicotinsäure und 4,6-Dimethoxy-2-methylsulfonylpyrimidin als Ausgangsstoffe, so läßt sich der Reaktionsablauf des erfindungsgemäßen Verfahrens durch das folgende Formelschema darstellen:

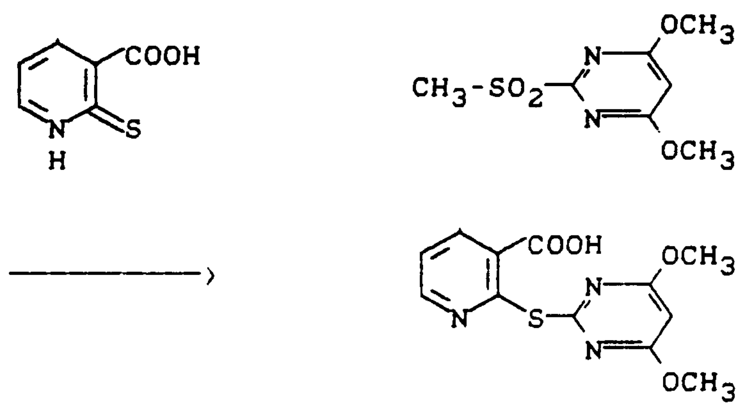

Die zur Durchführung des erfindungsgemäßen Verfahrens als Ausgangsstoffe benötigten Thiopyridoncarbonsäurederivate sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Thiopyridoncarbonsäurederivate der Formel (II) sind bekannt oder erhältlich in Analogie zu bekannten Verfahren (vgl. z.B. J. Heterocycl. Chem. 22, 1353 [1985]; EP 232067; Bull. Chem. Soc. Jpn. 49, 3274-3279 [1976]; JP 01275562 [1989]; Pr. Nauk. Akad. Ekon. Im. Oskara Langego Wroclawin 435, 149-155 [1988] bzw. CA 122 : 76911z; US 3850883; EP 4319).

Die zur Durchführung des erfindungsgemäßen Verfahrens weiterhin als Ausgangsstoffe benötigten Heterocyclen sind durch die Formel (III) allgemein definiert, In dieser Formel (III) stehen $R^3$, $R^4$, X und Z vorzugsweise für diejenigen Reste, die bereits im Zusammenhang mit der Beschreibung der erfindungsgemäßen Stoffe der Formel (I) als bevorzugt für diese Substituenten genannt wurden.

E steht vorzugsweise für Halogen oder Alkylsulfonyl, insbesondere für Chlor, Brom oder Methylsulfonyl. Die Heterocyclen der Formel (III) sind ebenfalls bekannt (vgl. z.B. PCT Int. Appl. WO 8704321; EP 94260; DE 2656183; J. Chem. Soc. C, 1966, 2031-2038, JP 63023870; EP 104876; DE 2230392; DE 2160780) oder erhältlich in Analogie zu bekannten Verfahren.

Als Verdünnungsmittel zur Durchführung des erfindungsgemäßen Verfahrens kommen inerte organische

Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethyl-ammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren wird vorzugsweise in Gegenwart eines geeigneten Reaktionshilfsmittels durchgeführt, Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden, Im allgemeinen arbeitet man bei Temperaturen zwischen 20°C und 180°C, vorzugsweise bei Temperaturen zwischen 60°C und 120°C.

Das erfindungsgemäße Verfahren wird bevorzugt unter Normaldruck durchgeführt. Es ist jedoch auch möglich unter erhöhtem Druck zu arbeiten.

Zur Durchführung des erfindungsgemäßen Verfahrens setzt man pro Mol an Thiopyridoncarbonsäurederivat der Formel (II) im allgemeinen 1.0 bis 2.0 Mol, vorzugsweise 1.0 bis 1.2 Mol an Heterocyclus der Formel (III) und gegebenenfalls 1.0 bis 3.0 Mol, vorzugsweise 1.0 bis 2.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum`

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono- und dikotyler Unkräuter insbesondere in monokotylen Kulturen wie beispielsweise

Weizen einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden, Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-

pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) sind möglich, Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden, Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab, Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

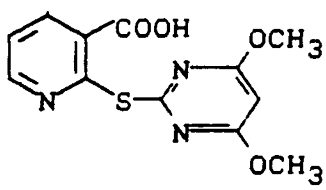

7.8 g (0.05 Mol) 2-Mercaptonicotinsäure (vgl. z.B. EP 4319), 10.9 g (0.05 Mol) 4,6-Dimethoxy-2-methylsulfonylpyrimidin (vgl. z.B. JP 63023870 [1988]) und 13.8 g (0.1 Mol) Kaliumcarbonat in 100 ml trockenem Dimethylformamid werden 18 Stunden bei 60 °C gerührt, danach auf Raumtemperatur abgekühlt, im Vakuum eingeengt, der Rückstand mit Wasser aufgeschlämmt, mit Salzsäure auf pH 2 eingestellt und mit Methyl-t-butylether extrahiert. Die vereinigten organischen Phasen werden über Natriumsulfat getrocknet, eingeengt, der Rückstand mit Diethylether verrührt, abgesaugt und getrocknet.

Man erhält 4.9 g (33% der Theorie) an 2-[2-(4,6-Dimethoxy)-pyrimidinylthio]-pyridin-3-carbonsäure vom Schmelzpunkt 149 °-152 °C.

Beispiel 2:

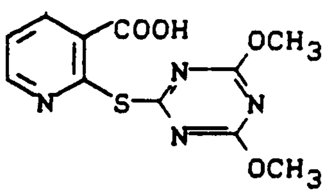

6.2 g (0.04 Mol) 2-Mercaptonicotinsäure, 7.0 g (0.04 Mol) 2-Chlor-4,6-dimethoxy-1,3,5-triazin (vgl. z.B. US 3316263) und 11.1 g (0.08 Mol) Kaliumcarbonat in 100 ml trockenem Dimethylformamid werden 18

Stunden bei 60 ° C gerührt, anschließend auf Raumtemperatur abgekühlt, mit Wasser versetzt, mit Salzsäure angesäuert, ausgefallener Niederschlag abgesaugt und getrocknet.

Man erhält 7.4 g (63% der Theorie) an 2-[2-(4,6-Dimethoxy)-1,3,5-triazinylthio]-pyridin-3-carbonsäure vom Schmelzpunkt 183 ° C (Zers.).

In entsprechender Weise erhält man folgende Beispiele:

Beispiel 3:

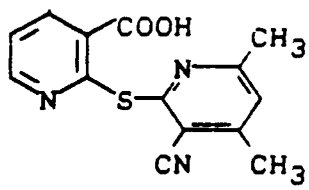

Fp. 183°C

| Beipiel-Nummer | Formel | physikalische Eigenschaften |
|---|---|---|
| 4 | 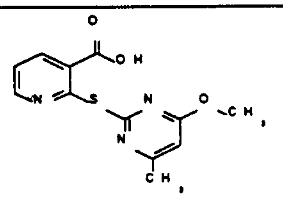 | Fp. 118-121°C |
| 5 | 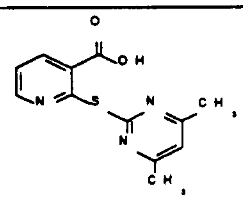 | Fp. >118°C (Zers.) |
| 6 | 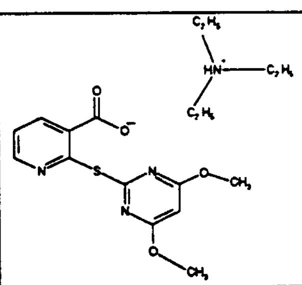 | $^1$H-NMR*): 5,7 (s, 1 H) |
| 7 | 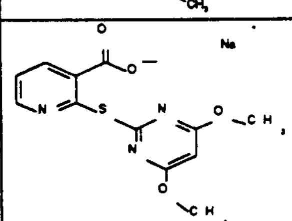 | $^1$H-NMR*): 6,0 (s, 1 H) |

| Beipiel-Nummer | Formel | physikalische Eigenschaften |
|---|---|---|
| 8 | 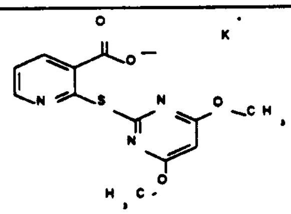 | $^1$H-NMR*): 6,0 (s, 1 H) |
| 9 | 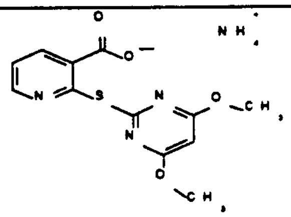 | $^1$H-NMR*): 6,0 (s, 1 H) |
| 10 | 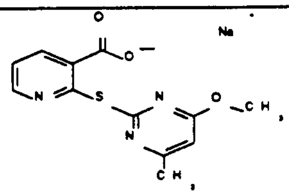 | $^1$H-NMR*): 6,55 (s, 1 H) |
| 11 | 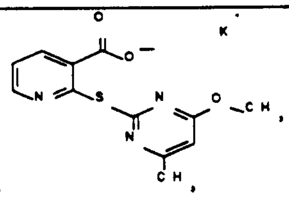 | $^1$H-NMR*): 6,55 (s, 1 H) |
| 12 | 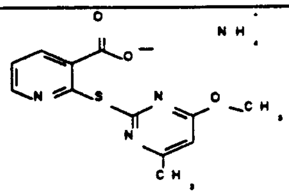 | $^1$H-NMR*): 6,6 (s, 1 H) |

| Beipiel-Nummer | Formel | physikalische Eigenschaften |
|---|---|---|
| 13 | 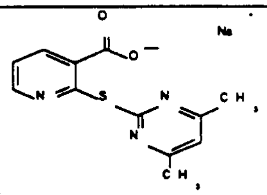 | $^1$H-NMR*): 7,05 (s, 1 H) |
| 14 | 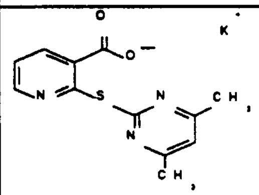 | $^1$H-NMR*): 7,05 (s, 1 H) |
| 15 | 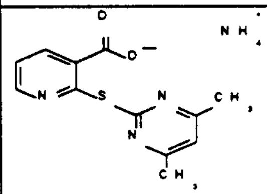 | $^1$H-NMR*): 7,1 (s, 1 H) |
| 16 | 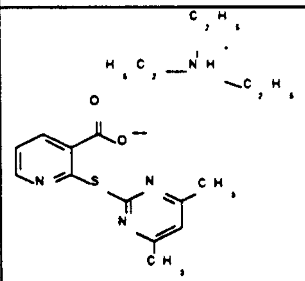 | $^1$H-NMR*): 6,8 (s, 1 H) |
| 17 | 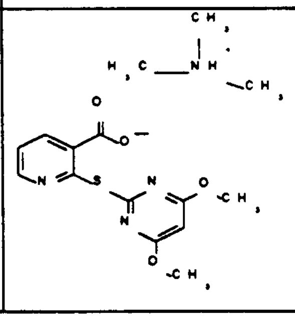 | $^1$H-NMR*): 5,7 (s, 1 H) |

| Beipiel-Nummer | Formel | physikalische Eigenschaften |
|---|---|---|
| 18 | 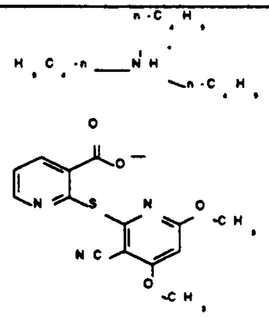 | $^{1}$H-NMR*): 5,7 (s, 1 H) |
| 19 | 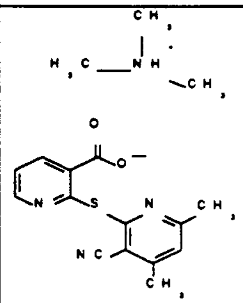 | $^{1}$H-NMR*): 7,1 (s, 1 H) |
| 20 | 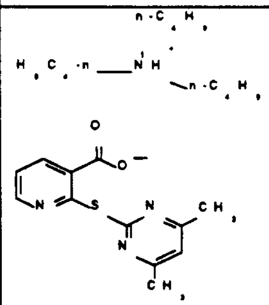 | $^{1}$H-NMR*): 7,05 (s, 1 H) |

| Beipiel-Nummer | Formel | physikalische Eigenschaften |
|---|---|---|
| 21 | 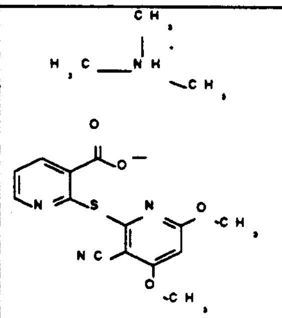 | $^1$H-NMR*): 7,4 (s, 1 H) |
| 22 | 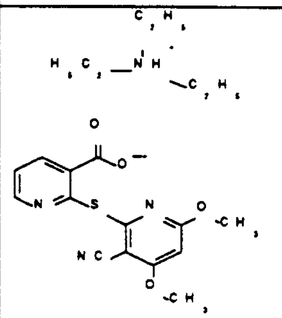 | $^1$H-NMR*): 7,1 (s, 1 H) |
| 23 | 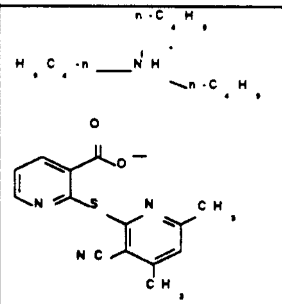 | $^1$H-NMR*): 7,1 (s, 1 H) |

*) Die $^1$H-NMR-Spektren wurden in Deuterochloroform ($CDCl_3$) oder in Hexadeuterodimethylsulfoxid (DMSO-$d_6$) mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichssubstanz eingesetzt:

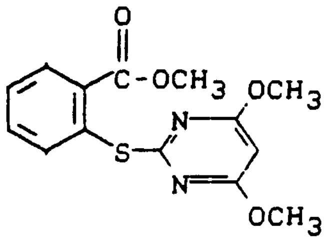

(A)

2-[2-(4,6-Dimethoxy)-pyrimidinylthio]-benzoesäuremethylester (bekannt aus EP 223406/Bsp. 11).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigt in diesem Test z.B. die Verbindung gemäß Herstellungsbeispiel 1.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigt in diesem Test z.B. die Verbindung gemäß Herstellungsbeispiel 1.

## Patentansprüche

**1.** Nicotinsäurederivate der allgemeinen Formel (I),

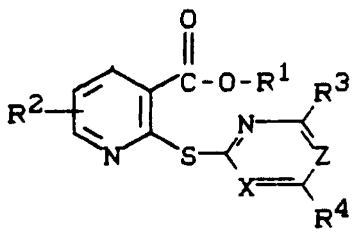

(I)

in welcher

| | |
|---|---|
| $R^1$ | für Wasserstoff, Alkyl, für ein Alkalimetallkation oder für ein gegebenenfalls substituiertes Ammoniumion steht, |
| $R^2$ | für Wasserstoff, Halogen, Alkyl, Alkoxy oder Halogenalkyl steht, |
| $R^3$ und $R^4$ | unabhängig voneinander jeweils für Wasserstoff, Alkyl, Alkoxy, Alkylthio, Halogenalkyl, Halogenalkoxy oder Halogenalkylthio stehen, |
| X | für Stickstoff oder für einen Rest C-$R^5$ steht und |
| Z | für Stickstoff oder für einen Rest C-$R^6$ steht, |

wobei

| | |
|---|---|
| $R^5$ | für Wasserstoff oder Cyano steht und |
| $R^6$ | für Wasserstoff oder Alkyl steht. |

**2.** Verbindungen der Formel (I) gemäß Anspruch 1, bei welchen

| | |
|---|---|
| $R^1$ | für Wasserstoff, für geradkettiges oder verzweigtes Alkyl mit 1 bis 4 Kohlenstoffatomen, für ein Lithium-, Natrium- oder Kalium-Kation oder für ein gegebenenfalls ein- bis dreifach gleich oder verschieden durch geradkettiges oder verzweigtes Alkyl mit 1 bis 4 Kohlenstoffatomen substituiertes Ammoniumkation steht, |
| $R^2$ | für Wasserstoff, Fluor, Chlor, Brom, Iod, für jeweils geradkettiges oder verzweigtes Alkyl oder Alkoxy mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht, |
| $R^3$ und $R^4$ | unabhängig voneinander jeweils für Wasserstoff, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen stehen, |
| X | für Stickstoff oder für einen Rest C-$R^5$ steht und |
| Z | für Stickstoff oder für einen Rest C-$R^6$ steht, |

wobei

| | |
|---|---|
| $R^5$ | für Wasserstoff oder Cyano steht und |
| $R^6$ | für Wasserstoff oder für geradkettiges oder verzweigtes Alkyl mit 1 bis 4 Kohlenstoffatomen steht. |

**3.** Verbindungen der Formel (I) gemäß Anspruch 1, bei welchen

| | |
|---|---|
| $R^1$ | für Wasserstoff, Methyl, Ethyl, n- oder i-Propyl oder für ein Lithium-, Natrium-, Kalium-, Ammonium-oder Triethylammoniumion steht, |
| $R^2$ | für Wasserstoff, Fluor, Chlor, Brom, Methyl, Ethyl, n- oder i-Propyl, Methoxy, Ethoxy, Trifluormethyl oder Trichlormethyl steht, |
| $R^3$ und $R^4$ | für Wasserstoff, Methyl, Ethyl, n- oder i-Propyl, Methoxy, Ethoxy, Methylthio, Ethylthio, Trifluormethyl, Trichlormethyl, Difluormethyl, Trifluormethoxy, Difluormethoxy, Trifluormethylthio, Trichlormethylthio, Difluorchlormethylthio oder Dichlorfluormethylthio stehen, |
| X | für Stickstoff oder eine C-CN-Gruppe steht und |
| Z | für Stickstoff oder eine C-$CH_3$- oder eine CH-Gruppe steht. |

**4.** Verfahren zur Herstellung von neuen Nicotinsäurederivaten der allgemeinen Formel (I),

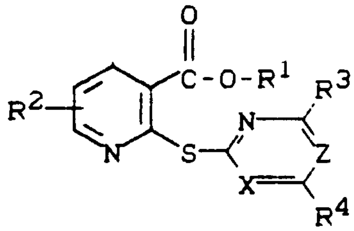

(I)

in welcher

| | |
|---|---|
| $R^1$ | für Wasserstoff, Alkyl, für ein Alkalimetallkation oder für ein gegebenenfalls substituiertes Ammoniumion steht, |
| $R^2$ | für Wasserstoff, Halogen, Alkyl, Alkoxy oder Halogenalkyl steht, |
| $R^3$ und $R^4$ | unabhängig voneinander jeweils für Wasserstoff, Alkyl, Alkoxy, Alkylthio, Halogenalkyl, Halogenalkoxy oder Halogenalkylthio stehen, |
| X | für Stickstoff oder für einen Rest C-$R^5$ steht und |
| Z | für Stickstoff oder für einen Rest C-$R^6$ steht, |

wobei

| | |
|---|---|
| $R^5$ | für Wasserstoff oder Cyano steht und |
| $R^6$ | für Wasserstoff oder Alkyl steht, |

dadurch gekennzeichnet, daß man Thiopyridoncarbonsäurederivate der Formel (II),

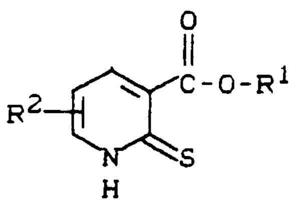

(II)

in welcher

$R^1$ und $R^2$ die oben angegebene Bedeutung haben,

mit Heterocyclen der Formel (III)

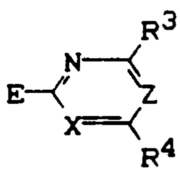

(III)

in welcher

| | |
|---|---|
| E | für eine elektronenanziehende Abgangsgruppe steht und |
| $R^3$, $R^4$, X und Z | die oben angegebene Bedeutung haben, |

gegebenenfalls in Gegenwart eines Verdünnungsmittels und gegebenenfalls in Gegenwart eines Reaktionshilfsmittels umsetzt.

**5.** Herbizide Mittel, gekennzeichnet durch einen Gehalt an mindestens einem Nicotinsäurederivat der Formel (I) gemäß den Ansprüchen 1 bis 4.

**6.** Verfahren zur Bekämpfung von Unkräutern, dadurch gekennzeichnet, daß man Nicotinsäurederivate der Formel (I) gemäß den Ansprüchen 1 bis 4 auf die Unkräuter und/oder ihren Lebensraum einwirken läßt.

7. Verwendung von Nicotinsäurederivaten der Formel (I) gemäß den Ansprüchen 1 bis 4 zur Bekämpfung von Unkräutern.

8. Verfahren zur Herstellung von herbiziden Mitteln, dadurch gekennzeichnet, daß man Nicotinsäurederivate der Formel (I) gemäß den Ansprüchen 1 bis 4 mit Streckmitteln und/oder oberflächenaktiven Substanzen vermischt.